# EUROPEAN PATENT APPLICATION

(11) **EP 3 338 562 A1**
(43) Date of publication of application: **27.06.2018**
(21) Application number: 17173860.2
(22) Date of filing: 31.05.2017
(51) Int. Cl.: A23L 3/26, A23L 3/3409, A61L 2/20

(54) **PROCESS AND APPARATUS FOR DECONTAMINATION OF FOOD ARTICLES**

(30) Priority: 20.12.2016 US 201662436777 P
(71) Applicant: Linde Aktiengesellschaft, 80331 München (DE)
(72) Inventor: NEWMAN, Michael D., Hillsborough, NJ 08844 (US); McCORMICK, Stephen A., Warrington, PA 18976 (US); MOLNAR, Istvan, 6230 Soltvadkert (HU)
(74) Representative: Hofmann, Andreas

(57) **Abstract**

In order to overcome the limitations and problems that earlier processes and apparatus have experienced, a process for decontamination of a food article (44) is proposed, comprising:
- generating an, in particular non-thermal, plasma (42) from a food grade gas, wherein the plasma (42) includes a vaporizable antimicrobial material; and
- exposing the food article (44) to the plasma (42).

A related apparatus (10) for decontamination of a food article (44) is also proposed.

## Description

### Technical field of the present invention

The present invention relates to a process and to an apparatus for decontamination of a food article using an, in particular non-thermal, plasma.

### Technological background of the present invention

The fruits, vegetables, and bakery markets are currently experiencing many issues with food bome illness and spoilage caused by contamination from various types of bacteria and mold spores.

Known methods to combat such contamination include disinfection with chemical sprays and washes using various ingredients (such as per-acetic acid as an active ingredient), and high pressure treatment of food products for bacteria kill.

One known method includes entraining a volatile antimicrobial agent such as a food acid or hydrogen peroxide in a carrier gas such as carbon dioxide or nitrogen, and contacting the food product with the mixture.

Non-thermal plasma (NTP), also known as cold plasma or non-equilibrium plasma, consists of gas molecules having moderate, near room or ambient temperatures, and electrons with higher temperatures, compared to thermal plasmas in which the electrons and gas temperatures are several thousands of degrees and are in equilibrium. The cold plasma discharge is weakly ionized, and therefore is effective for microbial decontamination without affecting the material being decontaminated.

Plasma is ionized gas that consists of a large number of different species such as electrons, positive and negative ions, free radicals, and gas atoms, molecules in the ground or excited state and quanta of electromagnetic radiation (photons).

It can be generated by means of coupling energy to a gaseous medium. In generating cold plasma, most of the coupled electrical energy is channeled to the electron component rather than heating the entire gas stream, such as by applying an electric field or electrical discharges to a neutral gas.

Power sources for cold plasma generation include microwave, radio frequency (RF), pulsed power, alternating current (AC) or direct current (DC) sources. Devices that have been used for plasma generation are AC voltage discharge, corona discharge, micro hollow cathode discharge, gliding arc discharge, one atmospheric uniform glow discharge, dielectric and resistive barrier discharge, dielectric plasma needle barrier discharge, and atmospheric pressure plasma jet.

Atmospheric, "cold" plasmas can be generated with minimal power, such as a few hundred watts of power and a supply of compressed gas, under room-temperature or ambient conditions in seconds to minutes, with little or no product heating. Examples of gases investigated for cold plasma decontamination of food articles include air, argon, helium, oxygen, nitrogen, and mixtures of these gases.

### Disclosure of the present invention: object, solution, advantages

Starting from the disadvantages and shortcomings as described above as well as taking the prior art as discussed into account, an object of the present invention is to overcome the limitations and problems that earlier processes and apparatus have experienced.

This object is accomplished by a process comprising the features of claim 1 as well as by an apparatus comprising the features of claim 7. Advantageous embodiments, expedient improvements and other optional features of the present invention are set forth herein and disclosed in the respective dependent claims.

There is provided herein a process for decontamination of a food article, comprising: generating a non-thermal plasma from a food grade gas, optionally carbon dioxide, wherein a vaporizable antimicrobial material is contained in the plasma, and exposing the food article to the plasma.

In the process according to the present invention, the vaporizable antimicrobial material may be introduced into the food grade gas prior to generation of the plasma.

In the process according to the present invention, the vaporizable antimicrobial material may be introduced into the generated plasma.

In the process according to the present invention, the antimicrobial material may be a food grade acid.

In the process according to the present invention, the food acid may be selected from the group consisting of carbonic acid, acetic acid, lactic acid, and mixtures thereof.

There is further provided herein an apparatus for decontamination of a food article comprising: a source of food grade gas, optionally carbon dioxide, in communication with a mixing vessel; a source of antimicrobial material; a metering pump for delivering antimicrobial material to the mixing vessel; optionally a holding chamber in communication with the mixing vessel and optionally having a sensor to detect the concentration of the antimicrobial material in the gas for feedback to the metering pump; a plasma generator in communication with the mixing vessel or the optional holding chamber; a process control valve for releasing the antimicrobial material in gas mixture to the plasma generator; and optionally a treatment chamber for applying a plasma generated from the mixture to a food article.

There is further provided herein an apparatus for decontamination of a food article comprising: a source of food grade gas, optionally carbon dioxide, in communication with a mixing vessel; a source of antimicrobial material also in communication with the mixing vessel; means for delivering antimicrobial material to the mixing vessel; optionally a holding chamber in communication with the mixing vessel and optionally having a sensor to detect the concentration of the antimicrobial material in the gas for feedback to the means for delivering; a plasma generator in communication with the mixing vessel or the optional holding chamber; means for releasing the antimicrobial material in gas mixture to the plasma generator; and means for applying a non-thermal plasma generated from the mixture to a food article.

There is further provided herein an apparatus for decontamination of a food article, comprising: a source of food grade gas, in particular of carbon dioxide, in communication with a mixing vessel; a source of antimicrobial material; means for delivering the antimicrobial material to the mixing vessel; a plasma generator in communication with the mixing vessel; and means for releasing the antimicrobial material in gas mixture to the plasma generator.

In the apparatus according to the present invention, a holding chamber in communication with the mixing vessel may be provided.

In the apparatus according to the present invention, the plasma generator may be in communication with the holding chamber.

In the apparatus according to the present invention, the holding chamber may have a sensor to detect the concentration of the antimicrobial material in the gas for feedback to the means for delivering.

In the apparatus according to the present invention, means for applying an, in particular non-thermal, plasma generated from the mixture to the food article may be provided.

In the apparatus according to the present invention, the means for applying may be a treatment chamber.

In the apparatus according to the present invention, the source of antimicrobial material may be in communication with the mixing vessel.

In the apparatus according to the present invention, the means for delivering may be a metering pump.

In the apparatus according to the present invention, the means for releasing may be a process control valve.

### Brief description of the drawings

For a more complete understanding of the present embodiment disclosures and as already discussed above, there are several options to embody as well as to improve the teaching of the present invention in an advantageous manner. To this aim, reference may be made to the claims dependent on claim 1 as well as on claim 7; further improvements, features and advantages of the present invention are explained below in more detail with reference to a particular and preferred embodiment by way of non-limiting example and to the appended drawing figure taken in conjunction with the following description of exemplary embodiments, of which:

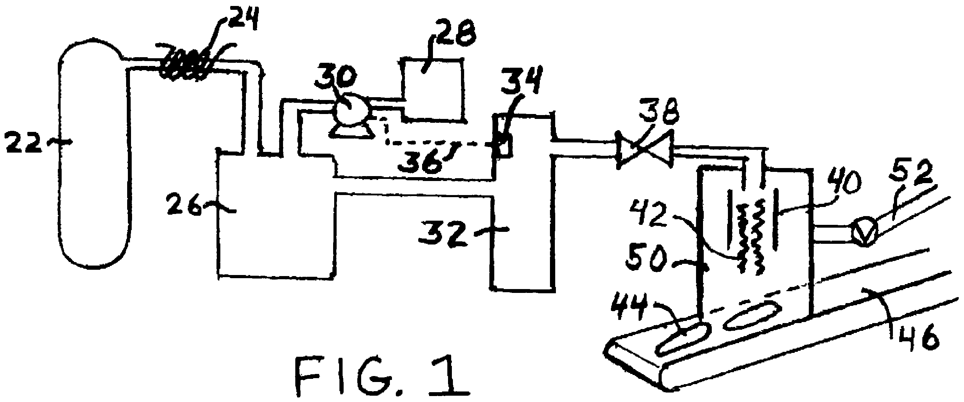

FIG. 1 shows a schematic diagram of an embodiment of an apparatus according to the present invention, said apparatus carrying out an embodiment of the process according to the present invention.

The accompanying drawing is included to provide a further understanding of the process and apparatus provided herein and is incorporated in and constitutes a part of this specification. The drawing illustrates an embodiment of the process and apparatus provided herein and, together with the description, serves to explain the principles described herein but is not intended to limit the specification or any of the claims.

### Detailed description of the drawings;

### best way of embodying the present invention

Before explaining the present inventive embodiment in detail, it is to be understood that the embodiment is not limited in its application to the details of construction and arrangement of parts illustrated in the accompanying drawing, since the present invention is capable of other embodiments and of being practiced or carried out in various ways. Also, it is to be understood that the phraseology or terminology employed herein is for the purpose of description and not of limitation.

In the following description, terms such a horizontal, upright, vertical, above, below, beneath and the like, are used solely for the purpose of clarity illustrating the present invention and should not be taken as words of limitation. The drawings are for the purpose of illustrating the present invention and are not intended to be to scale.

The present embodiments are directed to a process for decontamination of a food article comprising generating a non-thermal plasma from a food grade gas, such as carbon dioxide, wherein a vaporizable antimicrobial material is introduced into, or the vaporized antimicrobial material is contained in the plasma, and exposing the food article to the plasma.

The non-thermal or cold plasma containing the antimicrobial material is used to kill bacteria or mold spores on fruits and vegetables (either at the beginning or at the end of the distribution chain), and on bakery products prior to packaging, by exposing the food product to, or contacting the food product with, the antimicrobial containing cold plasma.

In certain embodiments, the vaporizable antimicrobial material may be introduced into the gas, such as carbon dioxide, prior to generation of the plasma. In these embodiments, a food grade acid (such as carbonic, acetic, lactic, or other acid) may be vaporized into CO₂ carrier gas that is then formed into the non-thermal or cold plasma by one of the processes disclosed above. Another suitable vaporizable antimicrobial material may comprise hydrogen peroxide.

In other embodiments, the vaporizable antimicrobial material may be introduced into the generated plasma and vaporized. Carbon dioxide in any form, but optionally as a compressed liquid or gas, is used to generate the non-thermal or cold plasma as above, and the antimicrobial material may be introduced into the plasma prior to or during exposure of the food article to the plasma.

By forming a cold plasma from the mixture of CO₂, such as when used as a carrier gas, in combination with an active ingredient, such as but not limited to carbonic, acetic, lactic, or other food grade acid, the final gas plasma is more effective in killing bacteria and mold spores than a CO₂ - generated plasma or the active ingredient alone. Therefore, either higher kill rates of bacteria and mold spores may be achieved, or smaller quantities of active ingredient may be used to achieve the desired final kill rate of bacteria and mold spores.

While the use of CO₂ as a carrier gas for active ingredients such as carbonic or acetic acids is known for treating perishable materials and to control microbial infestation, the formation of a cold plasma from a CO₂/acid gas mixture has not been practiced nor its effectiveness defined.

The following example is set forth merely to further illustrate the subject process for decontamination of a food article using a non-thermal plasma generated from a CO₂/acid gas. The illustrative example should not be construed as limiting the subject matter in any manner.

In an illustrative example, the process may proceed as follows. CO₂ gas from a CO₂ gas source 22 is slightly heated, such as with a heating element 24 to a temperature of between 25°C and 50°C, after which it is introduced into a mixing chamber 26 where an active food grade acid is delivered from an active antimicrobial material source 28 (such as carbonic, acetic, lactic, or other acid) in fluid communication with the mixing chamber 26.

The antimicrobial material is metered by a metering pump 30 into the flow stream from the source 28, and is thoroughly mixed with and dissolved into the CO₂ gas.

The resulting CO₂/acid vapor is then moved from the mixing chamber 26 into a holding chamber 32 or tank in fluid communication therewith, where the acid concentration can be measured by a sensor 34 operatively associated with the chamber 32.

The sensor 34 generates a feedback loop signal 36 to control the metering pump 30 such that precise control can be obtained of the acid concentration in the final CO₂/acid vapor mixture.

The CO₂/acid vapor mixture is stored in the holding chamber 32 until process vapor is required for use in the decontamination process, at which time a process control valve 38 opens, allowing the CO₂/acid vapor mixture to be delivered from the chamber 32 into a cold plasma generator 40 in fluid communication therewith.

In passing through the cold plasma generator 40, the CO₂/acid vapor is transformed into a cold plasma 42, which is then applied to a food article 44 to be treated.

A plurality of the food articles 44 may be conveyed or carried by a conveyor 46 passing in close operational proximity to the cold plasma generator 40.

In one embodiment, the process would use a treatment chamber 50 constructed and arranged with a vacuum system 52 for evacuating air or a modified ambient atmosphere prior to application of the cold plasma 42 to the food article 44.

The treatment chamber 50 may be constructed as a housing or enclosure sized and shaped to receive the conveyor 46 passing therethrough.

In another embodiment, the process would allow injection of the cold plasma 42 directly onto the food article 44 without the need of an evacuation chamber.

Applying the CO₂/acid gas generated as the cold plasma 42 provides even and uniform distribution of the active ingredient on all surfaces of the food article 44 and therefore, will achieve homogeneous bacteria kill on the product surface.

As a comparison, known chemical washes and sprays used to decontaminate food articles may be flushed into the processing plant drain, thereby resulting in wastewater treatment issues.

Known methods using chemical washes and sprays result in a large amount of wasted chemical which is not a cost-effective use of same. In contrast, the CO₂/acid cold plasma process embodiments herein require only a small quantity of active ingredient to be used, resulting in little if any waste associated with the process embodiments.

The processing time required for bacteria kill using CO₂/acid cold plasma is roughly ten seconds and is easily accomplished using automation.

Other food grade gases suitable for use in the subject process and apparatus embodiments include but are not limited to air, argon, helium, oxygen, nitrogen, and mixtures of such gases.

It will be understood that the embodiments described herein are merely exemplary, and that one skilled in the art may make variations and modifications without departing from the spirit and scope of the present invention. All such variations and modifications are intended to be included within the scope of the present invention as described and claimed herein. Further, all embodiments disclosed are not necessarily in the alternative, as various embodiments of the present invention may be combined to provide the desired result.

### List of reference signs

- 10: apparatus
- 22: source of food grade gas, in particular of carbon dioxide (CO₂) gas
- 24: heating element
- 26: mixing chamber or mixing vessel
- 28: source of antimicrobial material
- 30: means for delivering antimicrobial material, in particular metering pump
- 32: holding chamber or tank
- 34: sensor
- 36: feedback loop signal
- 38: means for releasing antimicrobial material, in particular process control valve
- 40: plasma generator, in particular non-thermal plasma generator, for example cold or non-equilibrium plasma generator
- 42: plasma, in particular non-thermal plasma, for example cold or non-equilibrium plasma
- 44: food article
- 46: conveyor
- 50: means for applying plasma 42, in particular treatment chamber
- 52: vacuum system

## Claims

1. A process for decontamination of a food article (44), comprising:
- generating an, in particular non-thermal, plasma (42) from a food grade gas, wherein the plasma (42) includes a vaporizable antimicrobial material; and
- exposing the food article (44) to the plasma (42).

2. The process according to claim 1, wherein the food grade gas is carbon dioxide.

3. The process according to claim 1 or 2, wherein the vaporizable antimicrobial material is introduced into the food grade gas prior to generation of the plasma (42).

4. The process according to at least one of claims 1 to 3, wherein the vaporizable antimicrobial material is introduced into the generated plasma (42).

5. The process according to at least one of claims 1 to 4, wherein the antimicrobial material is a food grade acid.

6. The process according to claim 5, wherein the food grade acid is an acid selected from the group consisting of carbonic acid, acetic acid, lactic acid, and mixtures thereof.

7. An apparatus (10) for decontamination of a food article (44), comprising:
- a source of food grade gas (22), in particular of carbon dioxide, in communication with a mixing vessel (26);
- a source of antimicrobial material (28);
- means for delivering (30) the antimicrobial material to the mixing vessel (26);
- a plasma generator (40) in communication with the mixing vessel (26); and
- means for releasing (38) the antimicrobial material in gas mixture to the plasma generator (40).

8. The apparatus according to claim 7, further comprising a holding chamber (32) in communication with the mixing vessel (26).

9. The apparatus according to claim 8, wherein the plasma generator (40) is in communication with the holding chamber (32).

10. The apparatus according to claim 8 or 9, wherein the holding chamber (32) has a sensor (34) to detect the concentration of the antimicrobial material in the gas for feedback to the means for delivering (30).

11. The apparatus according to at least one of claims 7 to 10, further comprising means for applying (50) an, in particular non-thermal, plasma (42) generated from the mixture to the food article (44).

12. The apparatus according to claim 11, wherein the means for applying (50) is a treatment chamber.

13. The apparatus according to at least one of claims 7 to 12, wherein the source of antimicrobial material (28) is in communication with the mixing vessel (26).

14. The apparatus according to at least one of claims 7 to 13, wherein the means for delivering (30) is a metering pump.

15. The apparatus according to at least one of claims 7 to 14, wherein the means for releasing (38) is a process control valve.
